# EUROPEAN PATENT APPLICATION

(11) **EP 4 230 235 A2**
(43) Date of publication of application: **23.08.2023**
(21) Application number: 23175678.4
(22) Date of filing: 05.12.2019
(51) Int. Cl.: A61M 1/00, A61B 1/018, A61B 1/307

(54) **FLEXIBLE URETEROSCOPE WITH DEBRIS SUCTION AVAILABILITY**

(30) Priority: 20.12.2018 US 201862782581 P
(62) Divisional of application: 19828466.3
(71) Applicant: Boston Scientific Scimed, Inc., Maple Grove, MN 55311 (US)
(72) Inventor: TAH, Richard C., Milford, Massachusetts 01757 (US); RAUNIYAR, Niraj Prasad, Plymouth, Minnesota 55446 (US)
(74) Representative: Pfenning, Meinig & Partner mbB

(57) **Abstract**

A ureteroscope system includes a handle including a first port and a second port and an elongated shaft extending from the handle to a distal end and including a shaft lumen open at the distal end of the shaft. The shaft is be inserted through a bodily lumen to a target surgical site. The first port is in communication with a handle channel which is in communication with the shaft lumen and the second port is in communication with a working channel extending through the handle and the shaft lumen to the distal end of the shaft. The system also includes a fluid source connected to the first port and pumps fluid through the handle channel and the shaft lumen to irrigate the site and a vacuum source connected to the second port and applies suction through the working channel to dislodge debris from the site.

## Description

### Priority Claim

The preset disclosure claims priority to U.S. Provisional Patent Application Serial No. 62/782,581 filed December 20, 2018; the disclosure of which is incorporated herewith by reference.

### Field

Aspects of the present disclosure generally relate to medical devices and methods. Particular aspects relate generally to ureteroscopy devices and methods.

### Background

Flexible ureteroscopes are generally used to examine a patient's kidneys and may be incorporated with features to improve accessibility and patient comfort. Generally, ureteroscopes include a working channel through which various tools may be inserted into the kidney. This working channel enables the ureteroscopes to be used in conjunction with, for example, laser fibers and medical retrieval baskets to break up and retrieve biological and foreign material, including kidney stones, from the body. In a typical procedure, laser lithotripsy is performed to break stones into multiple smaller fragments which are collected within the calyx. Each fragment is then individually removed using a basket device inserted through the working channel of the ureteroscope. This process of removing stone fragments individually can be time consuming, requiring multiple passages of medical devices in and out of the patient to retrieve each fragment, and may result in trauma to the lining of the ureter.

In another procedure, a vacuum system is used in which the stone fragments are sucked out through the working channel of the ureteroscope. During this procedure, irrigation fluid is pumped into the kidney through an access sheath and is expirated out, with the fragmented stones, through the working channel of the ureteroscope. However, this method of vacuuming stone fragments requires the use of an additional external access sheath to introduce the fluids to the kidney. Yet, such an increase in the diameter of the apparatus inserted may cause trauma to the ureter.

### Summary

The present disclosure relates to a ureteroscope system, comprising a handle configured to remain outside of the body, the handle including a first port and a second port, an elongated shaft extending from the handle to a distal end and including a shaft lumen, the shaft lumen being open at the distal end of the shaft, the shaft being configured to be inserted through a bodily lumen to a target surgical site, wherein the first port is in communication with a handle channel which is in communication with the shaft lumen and the second port is in communication with a working channel extending through the handle and the shaft lumen to the distal end of the shaft; a fluid source connected to the first port and configured to pump fluid through the handle channel and shaft lumen to irrigate the target surgical site, and a vacuum source connected to the second port and configured to apply suction through the working channel to dislodge debris from the target surgical site through the shaft lumen.

In an embodiment, the system may further comprise an end cap coupled to the distal end of the elongated shaft, the end cap including a plurality of holes extending through a side wall thereof, the holes being in communication with an interior space of the end cap, the interior space being in communication with the shaft lumen.

In an embodiment, the working channel may extend through the end cap to a distal tip thereof.

In an embodiment, the handle channel may be a closed channel.

In an embodiment, the system may further comprise at least one sensor, the sensor transmitting sensor data relating to the target surgical site to the user.

In an embodiment, the sensor may be a pressure transducer configured to measure pressure within the target surgical site.

In an embodiment, the system may further comprise a third port in communication with the handle channel, the third port configured to receive at least one medical device therethrough.

In an embodiment, the vacuum source may be connected to the second port via tubing.

The present disclosure also relates to a ureteroscope system, comprising a handle configured to remain outside of the body, the handle including a first, second and third ports, the handle including an internal tube open to the first port, a working channel open to the second port and a fluid channel open to the third port, an elongated shaft extending from the handle to a distal end and including a shaft lumen, the shaft being configured to be inserted through a bodily lumen to a target surgical site, wherein the fluid channel is in communication with the shaft lumen, the internal tube and working channel extending through the shaft lumen to the distal end of the shaft, a vacuum source connected to the second port and configured to apply suction through the working channel to dislodge debris from the target surgical site through the shaft lumen, and a fluid source connected to the third port and configured to pump fluid through the handle channel and shaft lumen to irrigate the target surgical site.

In an embodiment, the first port may be sized and shaped to receive a medical device therethrough.

In an embodiment, the system may further comprise an end cap coupled to the distal end of the elongated shaft, the end cap including a plurality of holes extending through a side wall thereof, the holes being in communication with an interior space of the end cap, the interior space being in communication with the shaft lumen.

In an embodiment, the internal tube and the working channel may extend through the end cap to a distal tip thereof.

In an embodiment, the internal tube, fluid channel, and working channel may be closed channels.

In an embodiment, the system may further comprise at least one sensor, the sensor transmitting sensor data relating to the target surgical site to the user.

In an embodiment, the sensor may be a pressure transducer configured to measure pressure within the target surgical site.

### Brief Description

Fig. 1 shows a side view of a ureteroscope system according to an exemplary embodiment of the present disclosure;
Fig. 2 shows a perspective view of a distal end of a shaft of the system of Fig. 1 according to an exemplary embodiment of the present;
Fig. 3 disclosure shows a side cross-sectional view of the ureteroscope system of Fig. 1;
Fig. 4 shows a cross-sectional view of the distal end of the shaft of Fig. 3;
Fig. 5 shows a side view of a ureteroscope system according to a second exemplary embodiment of the present disclosure;
Fig. 6 shows a side cross-sectional view of the ureteroscope system of Fig. 5;
Fig. 7 shows a perspective view of a distal end of a shaft of the system of Fig. 5 according to an exemplary embodiment of the present disclosure; and
Fig. 8 shows a side view of the ureteroscope system of Fig. 5 with a handle cover removed.

### Detailed Description

The present disclosure may be further understood with reference to the following description and appended drawings, wherein like elements are referred to with the same reference numerals. The present disclosure relates to devices, systems and methods for the removal of debris within a lumen (e.g., within a living body). Exemplary embodiments describe a system including a ureteroscope device such as the Litho Vue ^{™} including multiple ports and channels for separate insertion of medical devices, irrigation and suctioning of debris from the body lumen. In some embodiments, the ureteroscope may be utilized to engage stones, stone fragments, and/or other objects to be removed from kidneys, ureters, and/or bladder. It should be noted that the terms "proximal" and "distal", as used herein, are intended to refer to a direction toward (proximal) and away from (distal) a user of the device (e.g. physician).

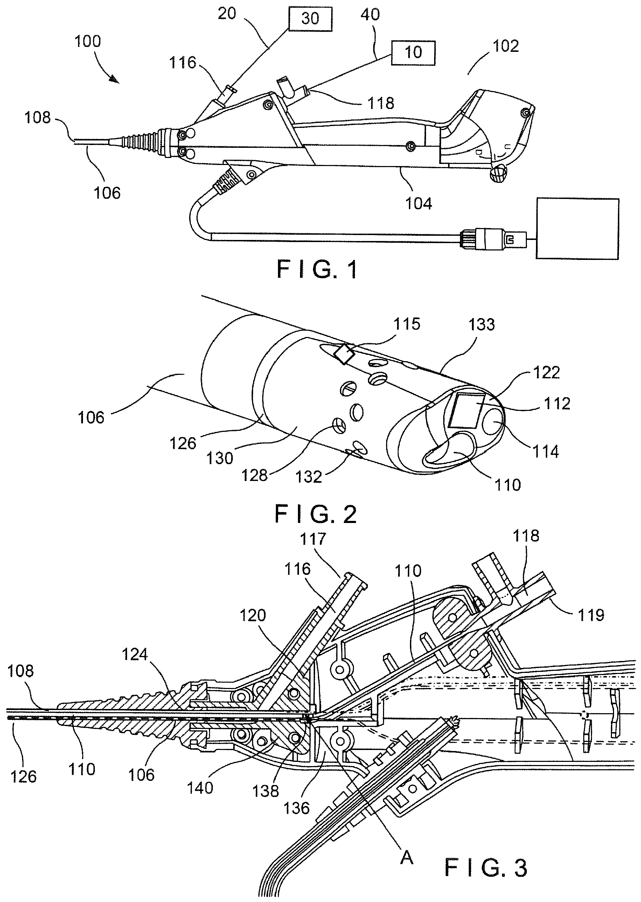

As shown in Figs. 1-4, a system 100 according to an exemplary embodiment of the present disclosure comprises a scope assembly 102 including a handle 104, which, during use, remains outside a living body, and a shaft 106 which is inserted into the body to a target site. The shaft 106 provides access into a bodily lumen (e.g., along a tortuous path, for example, in a body lumen accessed via a natural body orifice). The scope assembly 102 may comprise any scope configured for use in minimally invasive procedures, such as a ureteroscope, for example, under the brand name Litho Vue^{™}. The scope assembly 102 includes an actuator which permits the user to control the distal end of the shaft 106 when the shaft 106 is in the body lumen. The scope assembly 102 also includes an actuator for a vacuum pump 10.

In an exemplary embodiment shown in Figs. 1-4, as noted above, the scope assembly 102 such as, for example, a ureteroscope, provides fluid to a target cavity to irrigate the target cavity via a first path and provides suction, via second path, to the target cavity to draw the fluid and any debris from the cavity. The scope assembly 102 includes a scope shaft 106 sized and shaped to be inserted through a body lumen to a target cavity. The shaft 106 has an inner diameter defining a lumen 108 and includes a separate working channel 110 extending through the lumen 108. The scope assembly 102 also includes the handle 104 with an actuation mechanism which allows the user to control and articulate the shaft 106 when maneuvering the scope through the body lumen. In an exemplary embodiment, the scope assembly 102 includes means for illuminating and visualizing the body lumen and any inner cavities of the body.

For example, the scope assembly 102, in this embodiment, includes a camera 112 and a light source 114, as shown in Fig. 2. The camera 112 and light source 114 may each be controlled manually via a button (or other similar mechanism) on the handle 104. In an exemplary embodiment, the scope assembly 102 may include at least one sensor 115 incorporated therein. For example, in one embodiment, the scope assembly 102 may include a pressure sensor (transducer) 115 at a distal end 126 of the shaft 106 or positioned on an end cap 130 to measure pressure within, for example, the kidney. In an embodiment, the scope assembly 102 is connected to a vacuum source 10 via a supply line 20 (i.e., tubing).

For example, the scope assembly 102 may be connected to a vacuum pump 10 which provides vacuum pressure through the tubing 20, the handle 104, and the working channel 110 of the shaft 106, as will be described in further detail below. The scope assembly 102 is further connected to a fluid source 30 via, for example, a supply line 40. The fluid source 30 provides fluid flow through the supply line 40, the handle 104 and the lumen 108 of the shaft 106, as will be described in further detail below. The fluid flow and vacuum pressure may be controlled by a fluid flow on/off button (not shown) and a vacuum on/off button (not shown), respectively, on the handle 104. Thus, the user can turn the vacuum on when debris, fluid, etc., is within the target lumen and can turn the vacuum off when suction is unnecessary. Similarly, the user can control fluid to flow through the scope assembly 102 and into the target lumen to break up and flush out debris or stones therein but can turn the fluid flow off when unnecessary.

The handle 104 of this embodiment includes a first port 116 and a second port 118. In the present embodiment, as can be seen in Figs. 1 and 3, the first and second ports 116, 118 are separate ports including separate hubs 117, 119, respectively. However, one skilled in the art will understand that the ports 116, 118 can be designed as a single hub connected to two separate channels. The first port 116 is in communication with a first handle channel 120, which is in communication with the lumen 108 of the shaft 106. As can be seen in Figs. 3-4, the lumen 108 extends from a proximal end 124 of the shaft 106 to a distal end 126 and is communication with an interior space 128 of an end cap 130 positioned on the distal end 126 of the shaft 106. The end cap 130 includes a plurality of holes 132 open to the interior space 128 of the end cap 130, as will be described in further detail below. Thus, the scope assembly 102 allows for fluid flow from the fluid source to the first port 116 (via the first hub 117), through the handle 104 and shaft 106, and out into the body cavity via the end cap 130.

The second port 118 is in communication with the working channel 110 which extends from the second port 118, through the handle 104 and shaft 106 to the end cap 130, as shown in Fig. 3. The working channel 110 is open to an exterior of the scope assembly 102 at a distal tip 122 of the end cap 130, as can be seen in Fig. 2. The working channel 110, in this embodiment, is a separate channel from the first handle channel 120 and the lumen 108 of the shaft 106. That is, the working channel 110 is a closed channel with a distal portion that extends through the lumen 108 of the shaft 106 so that inclusions and medical devices (i.e., budding cables, illumination fibers or camera cables) inserted therethrough are sealed off from fluid passing through the lumen 108.

Each of the working channel 110 and the first handle channel 120 pass through an internal hub 140 within an interior cavity 136 of the handle 104. The hub 140 provides a fluid seal between the working channel 110/shaft 106 and the interior cavity 136. Specifically, the hub 140 includes a fluid seal 138 at point A, depicted in Fig. 3, which completely seals an interior portion of the hub 140 (through which the working channel passes 110) from the interior cavity 136 so that no fluid can enter into the handle cavity 136 through this point. The seal may be any conventional seal that surrounds a circumference of the working channel 110 at the entrance point A of the working channel 110 through the hub 140. Thus, the fluid passing through the scope assembly 102 is contained to the specific fluid flow pathway described above and backflow into the internal components of the handle 104 is prevented.

The second port 118 is configured to be coupled to the vacuum pump 10 so that debris and/or fluid within the target body cavity may be expirated through the working channel 110 as desired. This separation of channels (i.e., the working channel 110, the handle channel 120) simplifies the medical procedure being performed, reducing procedure time. Furthermore, the use of the inner diameter of the shaft 106 (i.e., the lumen 108) as a separate channel for providing irrigation fluid allows the shaft 106 to maintain a minimal profile for both irrigation and vacuuming without the use of a secondary, external channel, such as an access sheath.

The end cap 130, as depicted in Fig. 2, is configured to be coupled to the distal end 126 of the shaft 106. As noted above, the end cap 130 includes an interior space 128 or diameter which is in communication with the lumen 108 of the shaft 106. The end cap 130 includes the plurality of holes 132 extending through a side wall 133 thereof such that the interior space 128 is open to an exterior of the scope assembly 102, allowing fluid flow from the lumen 108, through the holes 132 and into the body cavity. As can be seen in the figure, the working channel 110 is configured to extend through the end cap 130 to a distal tip 122 thereof. Furthermore, the camera 112 and light source 114, in this embodiment, are positioned at the distal tip 122 of the end cap 130 while the transducer 115 is positioned at a side wall thereof. It will be understood by those skilled in the art, however, that the sensors 112, 115 and light source 114 may be positioned anywhere on a distal end of the scope assembly 102 including, for example, at the distal end 126 of the shaft 106.

In an embodiment, the system 100 includes a processing device 50, such as a computer. The processing device 50 is operatively connected to one or more system components such as, for example, the scope assembly 102, the vacuum pump 10 and the fluid source 30. The processing device 50 is capable of performing various functions such as calculation, control, computation, etc. For example, the processing device 50 may receive signals or data from sensors of the system 100 - i.e., camera 112 or the pressure transducer 115 - and determine from the data provided when and if the fluid source 30 or the vacuum source 10 should be turned on.

The processing device 50 may also be configured to include image recognition software that can detect visual noise from the camera 112. Thus, when the operator has identified a target item of debris to be broken up from the images, the operator may turn the fluid source 30 on to break up the target item of debris and suction the broken pieces into the device. The system may then automatically monitor the images (using the image recognition software) to determine when the images reach a degree of clarity or sharpness that indicates that the debris has been sufficiently cleared from the body lumen. At this point, the supply of fluid and suction may be terminated automatically. In another example, if the pressure transducer 115 at the distal tip 122 of the end cap 130 detects an increase in pressure within the cavity during suction of the debris, the system 100 may turn on the fluid source 30 to flush out any blockages that may have been caused by debris.

An exemplary method for removing debris from a body cavity includes inserting the distal end 126 of the shaft 106 into a target lumen and advancing the shaft 106 therethrough to a target cavity within, for example, the kidney. In some embodiments, irrigation fluid may be provided through the first port 116, the lumen 108 of the shaft 106, and into the target lumen. Once the shaft 106 has been positioned at a target site within the kidney, laser fibers and/or a basket device are inserted through the second port 118 and advanced through the working channel 110 until a distal end thereof extends past the distal end 126 of the shaft 106 and the end cap 130. As the medical device is advanced into the target lumen, sensors such as the pressure transducer 115 and the camera 112 provide feedback to the user and/or processing device 50 regarding conditions of the target site at which the scope assembly 102 is positioned, which may be displayed on a display screen.

During, for example, a lithotripsy, a kidney stone is broken up into multiple fragments and the scope assembly 102 is used to suction the fragments of the broken stone through the working channel 110 of the shaft 106. The pressure transducer 115 and the camera 112 monitor the breaking up of the fragments and allow the user to determine if irrigation fluid should be applied to the target cavity or if there is a need for further medical instruments to aid in the breaking up of the kidney stone. Once the debris has been fragmented, the vacuum source 10, which is connected to the second port 118, applies suction through the working channel 110 to vacuum the debris from the target cavity and out through the working channel 110 of the scope assembly 102. At any point, the user may manually turn off/on the fluid source 30, the vacuum source 10, and any sensors via use of the buttons on the handle 104.

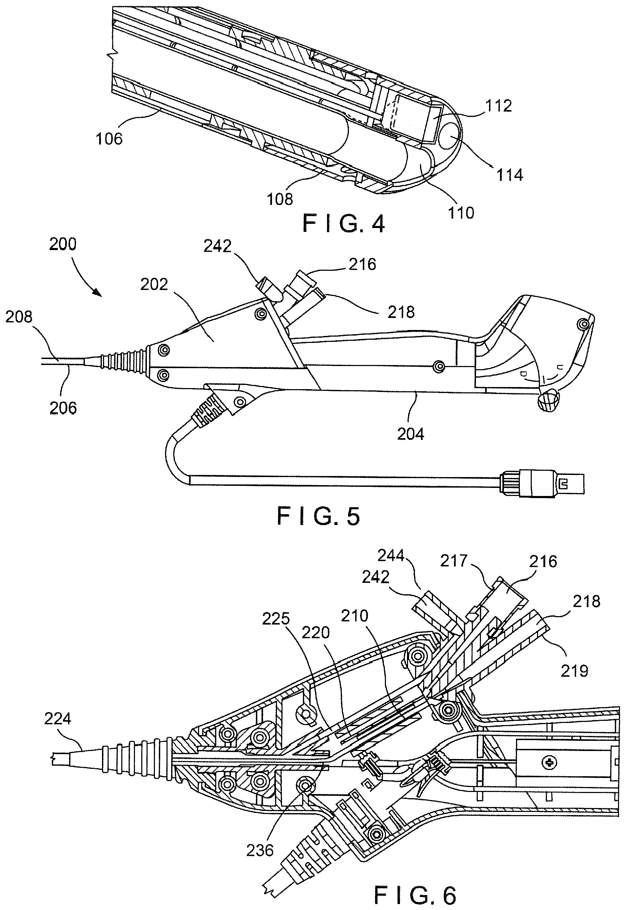

As shown in Figs. 5-8, a system 200 according to an exemplary embodiment of the present disclosure is substantially similar to the system 100 except as described herein. Specifically, the system 200 includes a scope assembly 202 with three ports instead of two. As can be seen in Fig. 5-6 and 8, a hub 217 of the first port 216 is combined with a hub 219 of the second port 218 and a third port 242 with a separate hub 244 is added to the scope assembly 202. The first port 216 is configured to receive a medical device while the second port 218 is configured for vacuuming debris from the target cavity and the third port 242 for pumping irrigation fluid to the target cavity. Thus, the ports 216, 218, 242 are separated to perform different functions during the procedure.

The first port 216, in this embodiment, extends from the hub 217 and is in communication with an internal first tube 220 that runs through the handle cavity 236 of the handle 204 and through the inner hub 240. The internal first tube 220, in turn, extends through the shaft 206 and exits at the distal tip of the end cap 230. As described above, the first port 216 is configured for the insertion of devices such as a laser fiber or a basket device through the scope assembly 202 to break up the kidney stone or debris found in the target cavity.

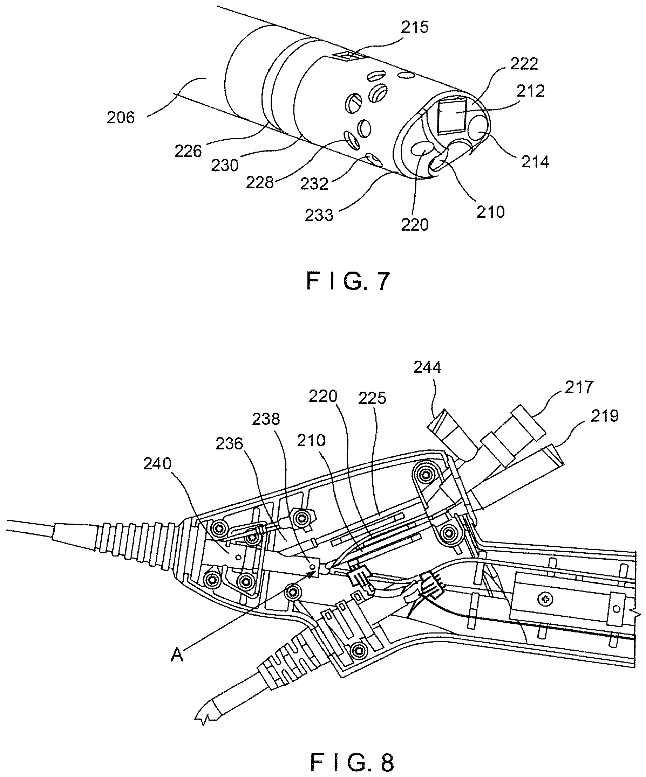

The second port 218, in this embodiment, is in communication with the working channel 210, which extends from the second port 218, through the hub 240 within the interior cavity 236 of the handle 204 and shaft 206, to the end cap 230, as shown in Fig. 6. The working channel 210 is open to an exterior of the scope assembly 202 at a distal tip 222 of the end cap 230, as can be seen in Fig. 7. The working channel 210, in this embodiment, similar to the working channel 110, is a separate channel from the internal first tube 220 and the lumen 208 of the shaft 206, as will be described in further detail below. As with the scope assembly 102, the hub 240 provides a fluid seal between the working channel 210 extending therethrough and the interior cavity 236 of the handle 204.

Specifically, a seal 238 at point A extends about a circumference of the working channel 210 to completely seal the interior of the hub 240, through which the working channel 210 passes, from the interior cavity 236. Thus, backflow of fluid is prevented. The working channel 210 is a closed channel with a distal portion that extends through the lumen 208 of the shaft 206 so that debris and fluid that are vacuumed through the working channel 210 do not interfere or come into contact with the medical devices that may be used in the procedure. Thus, the advantage of having a standalone working channel 210 is such that, for example, a laser fiber or other medical device can be inserted through the internal first tube 220 and, while fragmentation is being performed using these medical devices, debris can be vacuumed out simultaneously through the working channel 210 without requiring removal of the medical devices from the scope assembly 202.

The third port 242, in this embodiment, is in communication with the lumen 208 of the shaft 206 via a fluid channel 225 which extends through the handle 204. As can be seen in Figs. 6-7, the fluid channel 225 is a separate and closed channel that extends substantially parallel to the internal first tube 220 and the working channel 210. The fluid channel 225 extends from the hub 244 of the third port 242 to a proximal end of the shaft lumen 208. Similar to the lumen 108, the lumen 208 extends from a proximal end 224 of the shaft 206 to a distal end 226 and is in communication with an interior space 228 of the end cap 230 positioned on the distal end 226 of the shaft 206. The end cap 230 includes a plurality of holes 232 open to the interior space 228 of the end cap 130. Thus, the scope assembly 202 allows for fluid flow from the fluid source to the third port 242 (via the third hub 244), through the handle 204 and shaft 206, and out into the body lumen via the end cap 230.

The end cap 230 of this embodiment, as depicted in Fig. 7, is similarly configured to be coupled to the distal end 226 of the shaft 206. As with the end cap 130, the end cap 230 includes an interior space 228 or diameter in communication with the lumen 208 of the shaft 206. The end cap 230 includes the plurality of holes 232 extending through a side wall 233 thereof such that the interior space 228 is open to the exterior of the scope assembly 202, allowing fluid flow from the lumen 208, through the holes 232 and into the body cavity. As can be seen in the figure, the working channel 210 is configured to extend through the interior space 228 end cap 230 to a distal tip 222 thereof. Furthermore, the internal first tube 220 also extends through the interior space 228 of the end cap 230 to the distal tip 222. A camera 212 and light source 214 are positioned at the distal tip 222 of the end cap 230 while a transducer 215 is positioned on the side wall 233. It will be understood by those skilled in the art, however, that the sensors 212, 215 and light source 214 may be positioned anywhere on a distal end of the scope assembly 202 including, for example, at the distal end 226 of the shaft 206.

As noted previously, the inclusion of three ports 216, 218, 242 in the scope assembly 202 separated the different functionalities of vacuum, irrigation and medical device insertion into three separate pathways through the scope assembly 202. Thus, there is no interference between the three pathways connected to the ports 216, 218, 242, which can be used simultaneously to make the procedure being performed more efficient.

It will be appreciated by those skilled in the art that the current devices and methods are not limited to the disclosed embodiments. For example, the disclosed systems 100, 200, 300 may be used in various other procedures such as, for example, hysteroscopies, cystoscopies, etc. Thus, the systems 100, 200, 300 are not limited to use with a ureteroscope but may be used with other devices such as cystoscopes, hysteroscopes or any other device with a shaft inserted into a body channel/lumen/cavity.

It will be appreciated by those skilled in the art that changes may be made to the embodiments described above without departing from the inventive concept thereof. It should further be appreciated that structural features and methods associated with one of the embodiments can be incorporated into other embodiments. It is understood, therefore, that this invention is not limited to the particular embodiments disclose, but rather modifications are also covered within the scope of the present invention as define by the appended claims.

The present subject-matter includes, inter alia, the following aspects:
1. A ureteroscope system, comprising:
   a handle configured to remain outside of the body, the handle including a first port and a second port;
   an elongated shaft extending from the handle to a distal end and including a shaft lumen, the shaft lumen being open at the distal end of the shaft, the shaft being configured to be inserted through a bodily lumen to a target surgical site, wherein the first port is in communication with a handle channel which is in communication with the shaft lumen and the second port is in communication with a working channel extending through the handle and the shaft lumen to the distal end of the shaft;
   a fluid source connected to the first port and configured to pump fluid through the handle channel and the shaft lumen to irrigate the target surgical site; and
   a vacuum source connected to the second port and configured to apply suction through the working channel to dislodge debris from the target surgical site through the shaft lumen.
2. The system of aspect 1, further comprising an end cap coupled to the distal end of the elongated shaft, the end cap including a plurality of holes extending through a side wall thereof, the holes being in communication with an interior space of the end cap, the interior space being in communication with the shaft lumen.
3. The system of aspect 2, wherein the working channel extends through the end cap to a distal tip thereof.
4. The system of any one of aspects 1 to 3, wherein the handle channel is a closed channel.
5. The system of any one of aspects 1 to 4, further comprising at least one sensor, the sensor transmitting sensor data relating to the target surgical site to the user.
6. The system of aspect 5, wherein the sensor is a pressure transducer configured to measure pressure within the target surgical site.
7. The system of any one of aspects 1 to 6, further comprising a third port in communication with the handle channel, the third port configured to receive at least one medical device therethrough.
8. The system of any one of aspects 1 to 7, wherein the vacuum source is connected to the second port via tubing.
9. A ureteroscope system, comprising:
   a handle configured to remain outside of the body, the handle including a first, second and third ports, the handle including an internal tube open to the first port, a working channel open to the second port and a fluid channel open to the third port;
   an elongated shaft extending from the handle to a distal end and including a shaft lumen, the shaft being configured to be inserted through a bodily lumen to a target surgical site, wherein the fluid channel is in communication with the shaft lumen, the internal tube and working channel extending through the shaft lumen to the distal end of the shaft;
   a vacuum source connected to the second port and configured to apply suction through the working channel to dislodge debris from the target surgical site through the shaft lumen; and
   a fluid source connected to the third port and configured to pump fluid through the handle channel and the shaft lumen to irrigate the target surgical site.
10. The system of aspect 9, wherein the first port is sized and shaped to receive a medical device therethrough.
11. The system of either aspect 9 or 10, further comprising an end cap coupled to the distal end of the elongated shaft, the end cap including a plurality of holes extending through a side wall thereof, the holes being in communication with an interior space of the end cap, the interior space being in communication with the shaft lumen.
12. The system of aspect 11, wherein the internal tube and the working channel extend through the end cap to a distal tip thereof.
13. The system of any one of aspects 9-12, wherein the internal tube, fluid channel, and working channel are closed channels.
14. The system of any one of aspects 9 to 13, further comprising at least one sensor, the sensor transmitting sensor data relating to the target surgical site to the user.
15. The system of aspect 14, wherein the sensor is a pressure transducer configured to measure pressure within the target surgical site.

## Claims

1. A ureteroscope system (100), comprising:
a handle (104) configured to remain outside of the body, the handle (104) including a first port (116) and a second port (118);
an elongated shaft (106) extending from the handle (104) to a distal end and including a shaft lumen (108), the shaft lumen (108) being open at the distal end of the shaft (106), the shaft (106) being configured to be inserted through a bodily lumen to a target surgical site, wherein the first port (116) is in communication with a handle channel (120) which is in communication with the shaft lumen (108) and the second port (118) is in communication with a working channel (110) extending through the handle (104) and the shaft lumen (108) to the distal end of the shaft (106);
a fluid source (30) connected to the first port (116) and configured to pump fluid through the handle channel (120) and the shaft lumen (108) to irrigate the target surgical site; and
a vacuum source (10) connected to the second port (118) and configured to apply suction through the working channel (110) to dislodge debris from the target surgical site through the working channel (110).

2. The system (100) of claim 1, further comprising an end cap (130) coupled to the distal end of the elongated shaft (106), the end cap (130) including a plurality of holes (132) extending through a side wall (133) thereof, the holes (132) being in communication with an interior space (128) of the end cap (130), the interior space (128) being in communication with the shaft lumen (108).

3. The system (100) of claim 2, wherein the working channel (110) extends through the end cap (130) to a distal tip (122) thereof.

4. The system (100) of any one of claims 1 to 3, wherein the handle channel (120) is a closed channel.

5. The system (100) of any one of claims 1 to 4, further comprising at least one sensor (115), the sensor (115) transmitting sensor data relating to the target surgical site to the user, wherein the sensor (155) is preferably a pressure transducer configured to measure pressure within the target surgical site.

6. The system (100) of any one of claims 1 to 5, further comprising a third port in communication with the handle channel (120), the third port configured to receive at least one medical device therethrough, and/or wherein the vacuum source (10) is connected to the second port (118) via tubing.

7. The system (100) of any one of claims 1 to 6, wherein each of the working channel (110) and the handle channel (120) pass through an internal hub (140) within an interior cavity (136) of the handle (104), wherein the hub (140) provides a fluid seal between the working channel (110), the shaft (106) and the interior cavity (136).

8. The system (100) of claim 7, wherein the hub (140) includes a fluid seal (138) that surrounds a circumference of the working channel 110 at an entrance point (A) of the working channel (110) through the hub 140, wherein the fluid seal (138) seals an interior portion of the hub (140) through which the working channel passes (110) from the interior cavity (136) so that no fluid can enter into the handle cavity (136) through the entrance point (A).

9. A ureteroscope system (200), comprising:
a handle (204) configured to remain outside of the body, the handle (204) including a first, second and third ports (216, 218, 242), the handle (204) including an internal tube (220) open to the first port (216), a working channel (210) open to the second port (218) and a fluid channel (225) open to the third port (242);
an elongated shaft (206) extending from the handle (204) to a distal end and including a shaft lumen (208), the shaft (206) being configured to be inserted through a bodily lumen to a target surgical site, wherein the fluid channel (225) is in communication with the shaft lumen (208), the internal tube (220) and working channel (210) extending through the shaft lumen (208) to the distal end of the shaft (206);
a vacuum source connected to the second port (218) and configured to apply suction through the working channel (210) to dislodge debris from the target surgical site through the working channel (210); and
a fluid source connected to the third port (242) and configured to pump fluid through the fluid channel (225) and the shaft lumen (208) to irrigate the target surgical site.

10. The system (200) of claim 9, wherein the first port (216) is sized and shaped to receive a medical device therethrough.

11. The system (200) of either claim 9 or 10, further comprising an end cap (230) coupled to the distal end of the elongated shaft (206), the end cap (230) including a plurality of holes (232) extending through a side wall (233) thereof, the holes (232) being in communication with an interior space (228) of the end cap (230), the interior space (228) being in communication with the shaft lumen (208).

12. The system (200) of claim 11, wherein the internal tube (220) and the working channel (210) extend through the end cap (230) to a distal tip (222) thereof.

13. The system (200) of any one of claims 9-12, wherein the internal tube (220), fluid channel (225), and working channel (210) are closed channels, wherein the working channel (210), which extends through the shaft lumen (208), is a separate channel from the internal tube (220) and the shaft lumen (208).

14. The system (200) of any one of claims 9 to 13, further comprising at least one sensor (212, 215), the sensor (212, 215) transmitting sensor data relating to the target surgical site to the user, wherein the sensor (215) is preferably a pressure transducer (215) configured to measure pressure within the target surgical site.

15. The system of any one of claims 9 to 14, wherein the working channel (210) extends through an inner hub (240) within an interior cavity (236) of the handle (204), wherein the inner hub (240) provides a fluid seal between the working channel (210) extending therethrough and the interior cavity (236) of the handle (204).
